# EUROPÄISCHE PATENTSCHRIFT

(11) **EP 1 078 694 B1**
(45) Veröffentlichungstag und Bekanntmachung des Hinweises auf die Patenterteilung: **26.10.2005**
(21) Anmeldenummer: 00116578.6
(22) Anmeldetag: 01.08.2000
(51) Int. Cl.: B05B 11/02, A61M 11/02, B05C 11/02

(54) **Spender mit manuell betätigbarer Ausbringeinrichtung**
Manually activated dispenser
Distributeur à actionnement manuel

(30) Priorität: 25.08.1999 DE 19940236
(43) Veröffentlichungstag der Anmeldung: 28.02.2001
(73) Patentinhaber: Ing. Erich Pfeiffer GmbH, 78315 Radolfzell (DE)
(72) Erfinder: Ritsche, Stefan, 78315 Radolfzell (DE)
(74) Vertreter: Patentanwälte Ruff, Wilhelm, Beier, Dauster & Partner

(56) Entgegenhaltungen:
- WO-A-96/24439
- WO-A-98/30335
- WO-A-98/42447
- FR-A- 2 682 305
- US-A- 5 289 946
- US-A- 5 427 280

## Beschreibung

Aus der DE 197 53 147 A1 ist ein Spender zur Ausbringung, insbesondere zum Zerstäuben von Medien, insbesondere flüssigen Medikamenten, bekannt, der eine Charge des Medikaments in nur einem oder ggf. wenigen Hüben ausgibt. Die Betätigung erfolgt zwischen den Fingern des Benutzers, wobei der Daumen einen Betätigungsdrücker in Betätigungs- und Ausbringrichtung drückt.

In der EP-B-0 311 863 ist eine Austragsvorrichtung beschrieben, bei der ein Pumpenzylinder und ein nach Art einer Schnappverriegelung ausgebildeter federnder Anschlag so zusammenwirken, daß vor der Ausführung eines Teilhubes ein bestimmter Betätigungsdruck von dem Bediener aufgebracht werden muß, so daß nach Überwinden dieses Druckpunktes der Austrag der Flüssigkeit mit einer bestimmten Mindestkraft und Geschwindigkeit erfolgt. Diese Ausbildung stellt sicher, daß z.B. bei Zerstäubung des Mediums, von Beginn an ausreichender Zerstäubungsdruck vorhanden ist und daß die Pumpe bis zu ihrem Hubende betätigt wird und so den gesamten Inhalt des Medienspeichers, der gleichzeitig den Pumpenzylinder bildet, in einem oder zwei Hüben ausgibt. Solche Einmal- oder Mehrfachdosierer sind bedeutsam für die Ausgabe von Medikamenten, die bzgl. der Dosierung, Kontaminierung, Konservierung oder weiteren Kriterien besonders kritisch sind.

Die US 5 427 280 A beschreibt einen Spender, mit dem aufeinanderfolgend zwei Hübe durchführbar sind. Dazu ist eine Schlitzführung mit unterschiedlich langen Führungsbahnen vorgesehen, in denen ein Vorsprung läuft. Zwischen den Hüben wird ein Betätigungsdrücker durch eine Rückholfeder stets in die Betätigungsausgangslage zurückgeführt.

Die WO 96/24439 A beschreibt einen Einmal-Spender, bei dem der Betätigungsdrücker eine als Pumpzylinder dienende Ampulle enthält und der durch Sollbruch-Stellen eine Druckpunktsicherung aufweist, die eine Betätigung erst bei ausreichender Betätigungskraft sicherstellt. Bei einer Ausführung als Zweimal-Zerstäuber sind die Sollbruch-Ringe doppelt vorgesehen. Auch die WO 98/42447 zeigt eine solche Druckpunkteinrichtung, die jedoch durch einrastende Nuten und Ringrippen bewerkstelligt wird.

Die WO 98/30335 beschreibt eine Weiterentwicklung dieses Systems, bei dem der erste Hub durch einen festen Anschlag gegen versehentliches Durchbrechen beim ersten Hub gesichert ist.

Die US 5 289 946 beschreibt einen Mehrfach-Spender mit einem Zählmechanismus.

Die FR 2 682 305 A beschreibt einen Spender, bei dem der das auszugebende Medium enthaltende Spender in einem Gehäuse verschiebbar geführt und durch eine Feder in Hubrichtung belastet ist. Nach Vorspannung dieses Mechanismus' kann ein Austraghub durch Auslösung einer Federklinke bewirkt werden. Auch hier befindet sich die Vorrichtung im Ruhezustand in der Betätigungsausgangslage.

### AUFGABE UND LÖSUNG

Aufgabe der Erfindung ist es, derartige Spender so auszubilden, dass sie einerseits gegen versehentliche Betätigung gesichert und andererseits ein möglichst geringes Pack- bzw. Lagervolumen aufweisen.

Diese Aufgabe wird durch die Merkmale des Anspruchs 1 gelöst.

Dadurch, daß sich der Betätigungsdrücker anfangs in seiner Betätigungsendlage befindet, ist ein versehentliches Betätigen des Bestätigungsdrückers nicht möglich. Gleichzeitig wird der Packraum des Spenders um die Länge eines Betätigungshubes des Betätigungsdrückers verringert.

Besonders bevorzugt sind dabei Spender, die ein Gehäuse mit daran angeformter Griffläche zur Auflage wenigstens eines Fingers und mit einer Ausbringungsöffnung aufweisen, wobei der Betätigungsdrücker relativ beweglich zur Griffläche im Gehäuse geführt ist.

Vorteilhafterweise ist die Relativbewegung zwischen Betätigungsdrücker und Gehäuse eine Linearbewegung.

Weiter vorteilhaft ist es, wenn die Betätigungsendlage durch einen die Hubbewegung des Betätigungsdrückers begrenzenden ersten Anschlag festgelegt wird.

Ebenfalls vorteilhaft ist es, wenn die Betätigungsausgangslage der Hubbewegung durch einen zweiten einen der Hubbewegung entgegengesetzte Bewegungsrichtung begrenzenden Anschlag festgelegt wird.

Insbesondere vorteilhaft ist es, derartige Spender mit einer Ausbringeinrichtung zu versehen, die aus einer Schubkolbenpumpe besteht, deren Pumpenzylinder der Speicher für das auszubringende Medium ist, wobei in vorteilhafter Weiterbildung der Pumpenzylinder aus einer zylindrischen Glasampulle besteht, die in eine Hülse eingesetzt ist.

Die vorteilhaften Weiterbildungen der Erfindung gemäß den Ansprüchen 6 und 7 betreffen zwischen Pumpenzylinder und Gehäuse wirkende Rast bzw. zwischen Betätigungsdrücker und Pumpenzylinder wirkende Schleppmittel, die eine unerwünschte Bewegung des Pumpenzylinders während einer Bewegung in Gegenrichtung der betätigten Hubbewegung bzw. während eines Hubes der Pumpe verhindert.

Gemäß dem Anspruch 8 erfolgt die Bewegung des Betätigungsdrückers aus der Betätigungsendlage in die Betätigungsausgangslage durch manuelle Betätigung. Gemäß den Ansprüchen 9 und 10 erfolgt vorteilhafterweise die Bewegung des Betätigungsdrückers aus der Betätigungsendlage in die Betätigungsausgangslage mittels eines Kraftspeichers wobei der Kraftspeicher vorzugsweise in Betätigungsendlage vorgespannt gehalten wird, wobei die vorgespannte Haltelage durch Betätigen eines Löseelements gelöst werden kann und so die Bewegung des Betätigungsdrückers von der Betätigungsendlage in die Betätigungsausgangslage erfolgt. In weiterer Ausgestaltung ist es möglich, daß der Kraftspeicher in Betätigungsendlage (I) durch Haltemittel vorgespannt gehalten ist und die Haltemittel durch Betätigen eines Löseelements lösbar sind, wodurch eine Bewegung des Betätigungsdrückers (5) von der Betätigungsendlage (I) in die Betätigungsausgangslage (II) erfolgt und insbesondere bei oder vor dem ersten Betätigen des Löseelements eine Originalitätssicherung irreversibel zerstört wird.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung auf anderen Gebieten verwirklicht sein und vorteilhafte sowie für sich schutzfähige Ausführungen darstellen können, für die hier Schutz beansprucht wird. Die Unterteilung der Anmeldung in einzelne Abschnitte sowie Zwischen-Überschriften beschränken die unter diesen gemachten Aussagen nicht in ihrer Allgemeingültigkeit.

### KURZBESCHREIBUNG DER ZEICHNUNGEN

Ein Ausführungsbeispiel der Erfindung ist in den Zeichnungen dargestellt und wird im folgenden näher erläutert. Dies geschieht anhand eines Beispiels bei dem die Gesamtcharge in zwei Hüben ausgebracht wird. Die Figuren 1 und 2 zeigen einen Spender vor der ersten Betätigung, die Figuren 3a, 3b bis 7a, 7b zeigen jeweils im Schnittbild wie auch in teilgeschnittener perspektivischer Darstellung eine Abfolge von Stellungen, wie sie sich bei der zweihubigen Betätigung des Spenders ergeben. Im einzelnen zeigt:

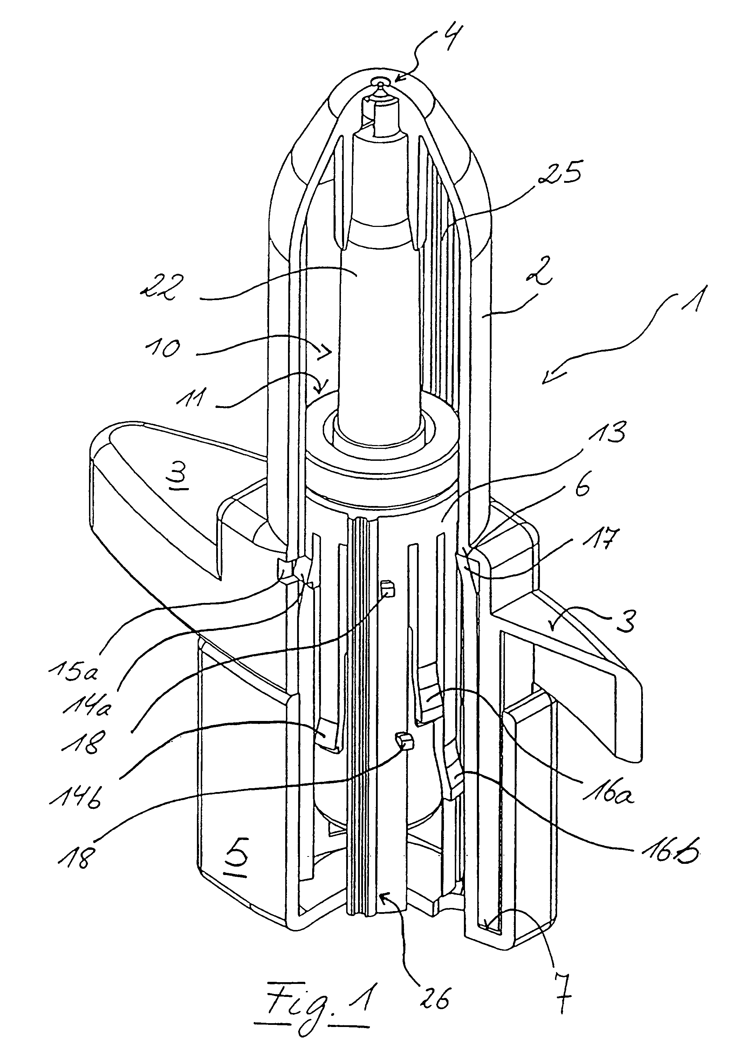

- Fig. 1: eine teilgeschnittene Perspektivdarstellung eines erfindungsgemäßen Spenders vor seiner ersten Betätigung;

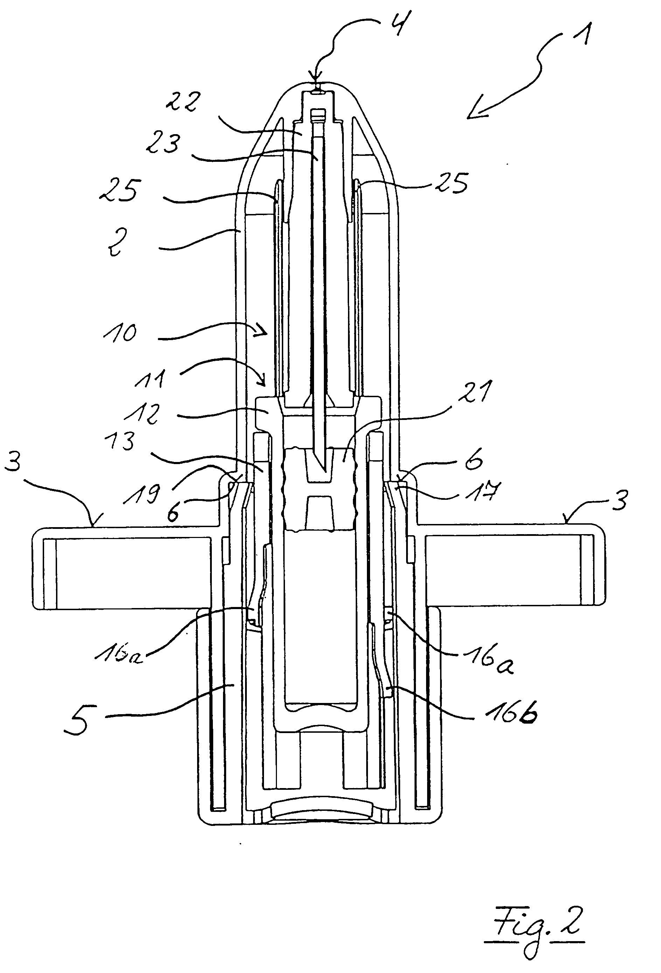

- Fig. 2: eine Schnittdarstellung durch einen Spender gemäß Fig. 1;

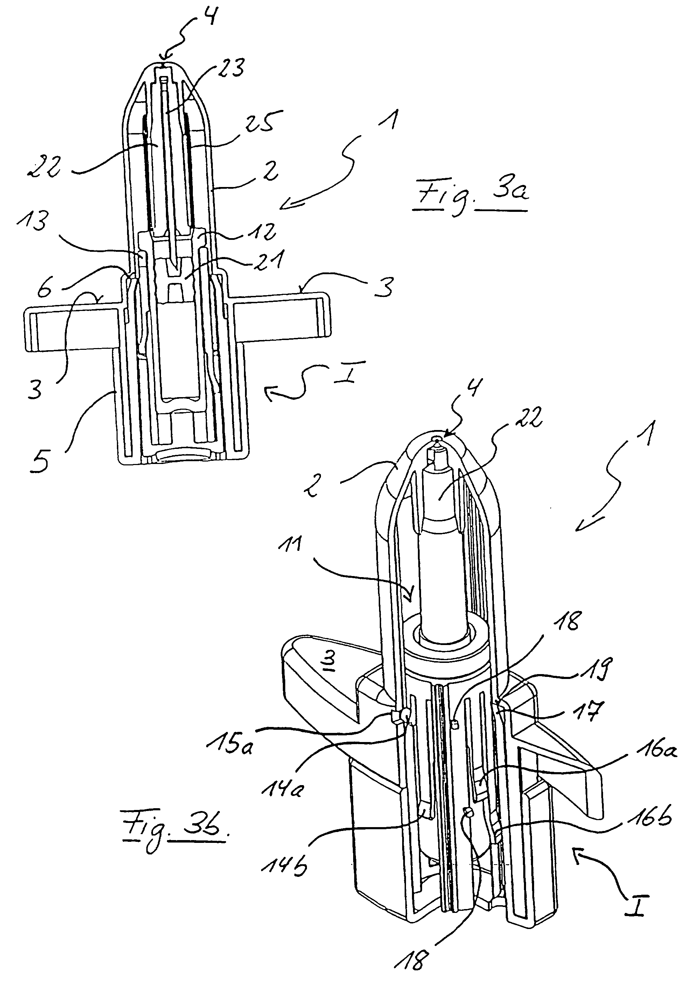

- Fig. 3a, 3b: die Stellung vor der ersten Betätigung;

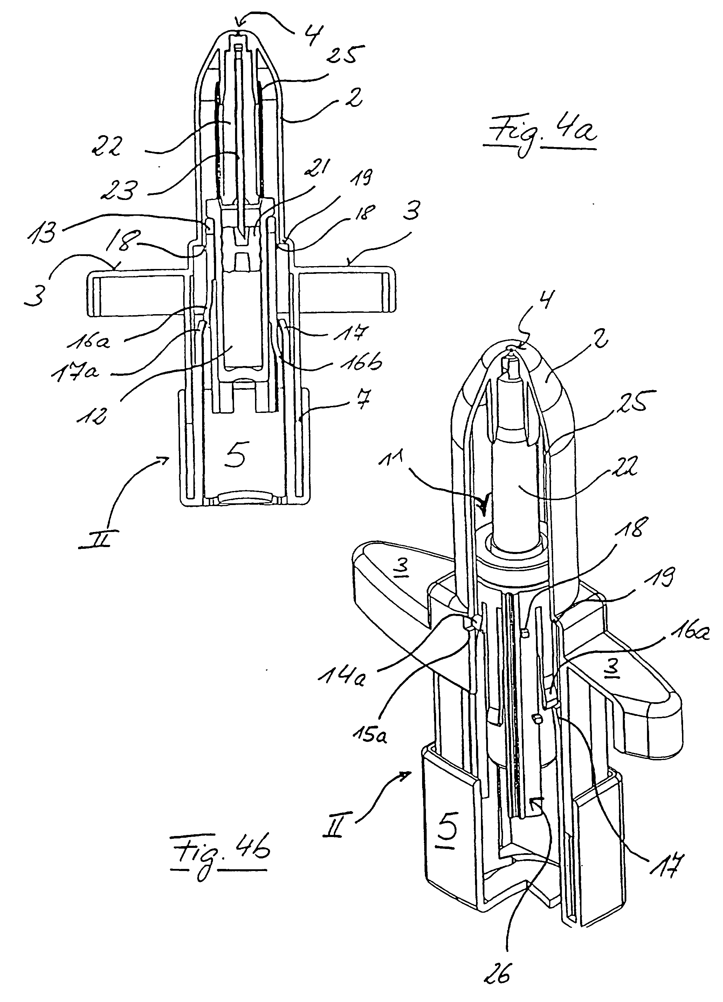

- Fig. 4a, 4b: die Stellung des Betätigungdrückers in Betäti- gungsausgangslage vor dem ersten Hub;

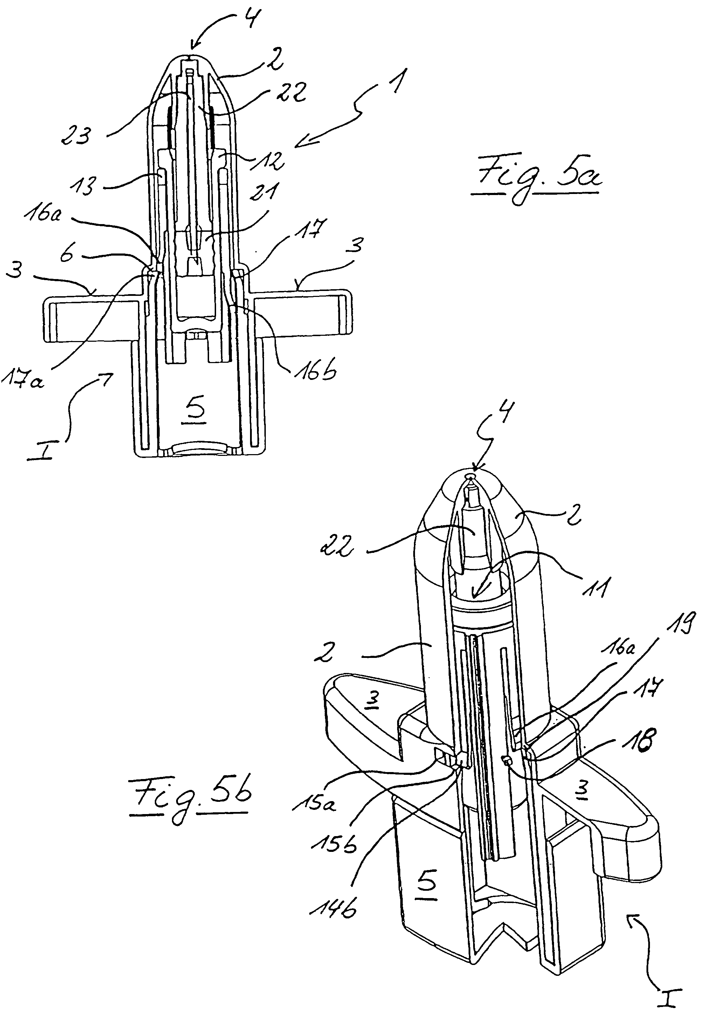

- Fig. 5, 5a: die Stellung des Betätigungsdrückers in Betätigungsendlage nach dem ersten Hub;

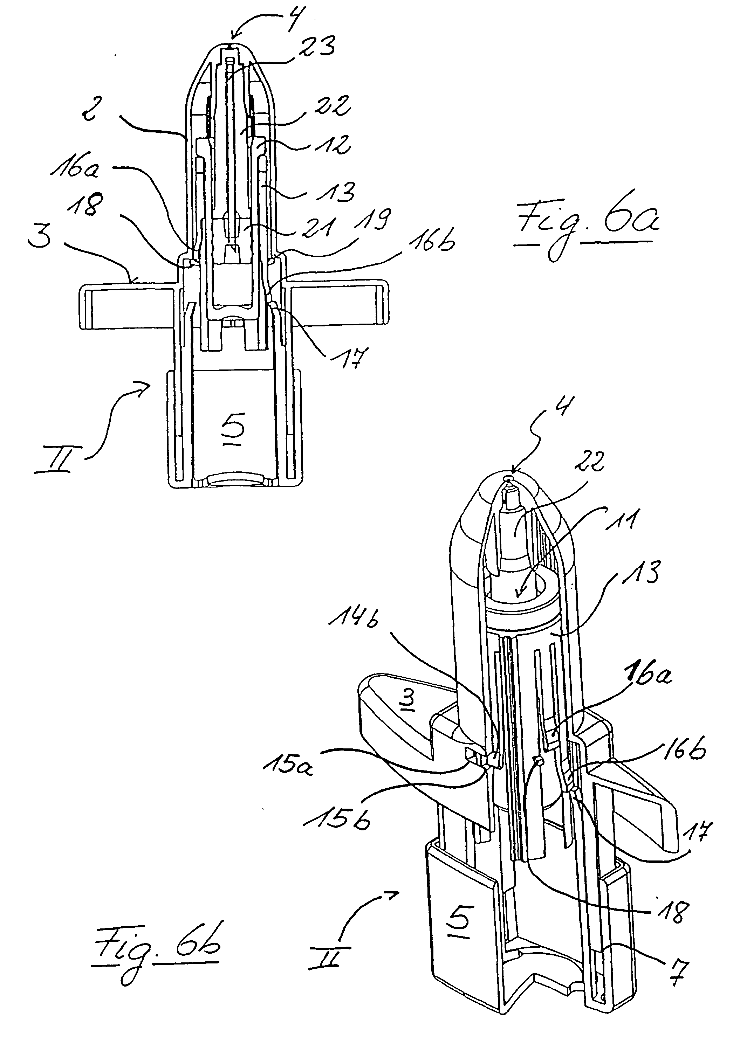

- Fig. 6, 6a: die Stellung des Betätigungsdrückers in Betätigungsausgangslage vor dem zweiten Hub und

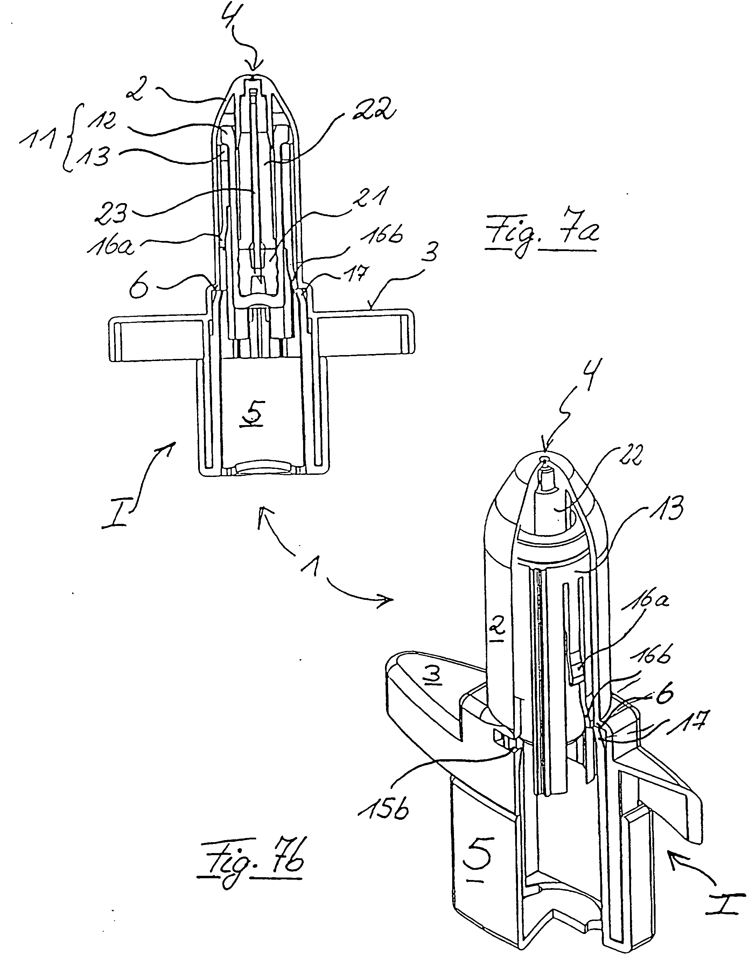

- Fig. 7a, 7b: die Stellung des Betätigungsdrückers in Betätigungsendlage nach dem zweiten Hub.

### BESCHREIBUNG DES AUSFÜHRUNGSBEISPIELS NACH FIG. 1 UND FIG. 2

Die Fig. 1 zeigt in perspektivischer, teilgeschnittener Darstellung, einen erfindungsgemäßen Spender, bei dem sich der Betätigungsdrücker 5 in der Betätigungsendlage vor dem ersten Hub befindet. Der Spender 1 hat ein Gehäuse 2, an dem Grifflächen 3 zur Auflage wenigstens eines Fingers angeformt sind. Als Ausbringeinrichtung ist in dem Gehäuse 2 eine Schubkolbenpumpe 10 angeordnet. Der Pumpenzylinder 11 der Schubkolbenpumpe 10 wird gebildet aus einer Glasampulle 12, die in einer Hülse 13 gehalten ist. Der Kolben der Schubkolbenpumpe wird vom Schaft 22 beaufschlagt. Die Hülse 13 weist Führungsschienen 26 auf, die in gehäuseseitigen Führungsnuten 25 gleiten und ein Verdrehen der Hülse 13 bzw. ein Verkanten zwischen dem Schaft 22 und dem Pumpenzylinder 11 verhindern. Zur Betätigung der Schubkolbenpumpe 10 dient der Betätigungsdrücker 5, der in axialer Richtung der Schubkolbenpumpe zwischen zwei Endlagen, die durch einen ersten Anschlag 6 und einen zweiten Anschlag 7 definiert sind, am Gehäuse 2 angeordnet ist. Der Betätigungsdrücker 5 weist ebenso wie das Gehäuse 2 in dem Bereich unterhalb der Griffläche 3 einen annähernd rechteckigen Querschnitt auf, wodurch eine verdrehsichere und verkantungsarme Führung des Betätigungsdrückers 5 am Gehäuse 2 erzielt wird. Ferner ermöglicht diese Gestaltung des Betätigungsdrückers 5 eine gute manuelle Betätigbarkeit. Ferner übermittelt sie dem Benutzer auf taktiler Basis Informationen über die Lage des Spenders.

Das gehäuseseitige Rastloch 15a bildet in Verbindung mit der hülsenseitigen Rastnocke 14a ein Rastmittel, das eine der Hubbewegung des Pumpenzylinders 11 entgegengesetzte Bewegung des Pumpenzylinders verhindert. Damit die Rastverbindung zwischen Gehäuse 2 und Hülse 13 bei einem Hub des Pumpenzylinders gelöst werden kann, ist die Rastnocke an einem Federsteg angeformt. Federsteg und Rastnocke sind dabei Teil der Hülse 13.

Während der Hubbewegung des Betätigungsdrückers 5 besteht eine Schleppverbindung zwischen dem Mitnehmer 17 des Betätigungsdrückers und dem Federsteg 16a, 16b, der an der Hülse 13 ausgebildet ist. Die Schleppverbindung zwischen Mitnehmer 17 und Federsteg 16b besteht während des zweiten Hubes und lediglich solange wie eine die Ausbringeinrichtung betätigende Hubbewegung des Betätigungsdrückers 5 erfolgt. An der Hülse 13 sind auch Druckpunktmittel 18 angeformt, wobei zwischen Druckpunktmittel 18 und Hülse 13 eine Sollbruchstelle besteht. Zweck der Druckpunktmittel 18 ist es, daß die Austragshübe mit jeweils einem vorbestimmten Mindestdruck erfolgen, wobei der Mindestdruck zum einen sicherstellt, daß ein Hub derart durchgeführt wird, daß dabei der Betätigungsdrücker 5 vollständig aus der Betätigungsausgangslage II in die Betätigungsendlage I überführt wird und zum anderen sicherstellt, daß an der Ausbringungsöffnung der erwünschte Sprühnebel austritt und dort geringe Wirkstoffverluste durch Tropfenbildung im Bereich der Ausbringöffnung entstehen.

Die Fig. 2 zeigt den Spender von Fig. 1 in einem Querschnitt. Der Spender 1 mit dem Gehäuse 2 und den am Gehäuse 2 angeformten Grifflächen und der im Gehäuse 2 ausgebildeten Ausbringöffnung 4 dient als Aufnahme der Schubkolbenpumpe 10. Die Schubkolbenpumpe 10 wird aus Pumpenzylinder 11, bestehend aus Hülse 13 und in der Hülse gehaltenen Glasampulle 12, sowie dem den Kolben 21 betätigenden Schaft 22. In dem Schaft 22 ist dabei eine Nadel 23 angeordnet. Der Kolben 21 ist zugleich der Verschlußstopfen der Ampulle 12. Er kann von der Nadel 23 durchstochen werden.

An dem Gehäuse 2 ist eine durch einen Absatz im Gehäuse gebildete Scherkante 19 ausgeformt, die dazu dient, die Druckpunktmittel 18 von der Hülse 13 abzuscheren, wenn eine Hubbetätigung des Betätigungsdrückers 5 mit mehr als der erforderlichen Mindestkraft erfolgt.

In Bezug auf die Längsachse der Schubkolbenpumpe 10 ist der Betätigungsdrücker 5 axial verschiebbar im Gehäuse 2 gelagert, wobei die am Gehäuse 2 angeformte Scherkante zugleich als die Betätigungsendlage definierender erster Anschlag 6 zwischen Betätigungsdrücker 5 und Gehäuse 2 bildet. Der die Betätigungsausgangslage sichernde zweite Anschlag 7 ergibt sich durch eine Begrenzung des Verschiebeweges des Betätigungsdrückers im Gehäuse. Radial gesehen zwischen Glasampulle 12 und Innenseite des Betätigungsdrückers 5 befindet sich die Hülse 13. Die überdrückbaren Federstege 16a, 16b gleiten entlang der Innenseite des Betätigungsdrückers 5. Zum Herstellen der Schleppverbindung zwischen Pumpenzylinder 11 mit der Hülse 13 und dem Betätigungsdrücker 5 dient der am Betätigungsdrücker 5 angeformte Mitnehmer 17.

### BESCHREIBUNG DER FUNKTION ANHAND DER FIG. 3a, 3b BIS FIG. 7a, 7b

Die Fig. 3a zeigt den Querschnitt durch einen erfindungsgemäßen Spender während die Fig. 3b eine teilgeschnittene Perspektivdarstellung des Spenders zeigt. Beidesmal befindet sich der Betätigungsdrücker 5 in der Betätigungsendlage I, jedoch hat noch kein Hub stattgefunden, bei dem ein Teil der Charge oder die ganze Charge ausgebracht worden wäre. In dieser dargestellten Stellung ist der Spender 1 gegen versehentliches Betätigen geschützt. Ferner weist er einen geringen Platzbedarf auf und kann so mit geringem Packvolumen verpackt und aufbewahrt werden.

Die Betätigungsendlage I des Betätigungsdrückers 5 wird durch den ersten Anschlag 6 zwischen Gehäuse 2 und Betätigungsdrükker 5 definiert. Fener besteht zwischen Betätigungsdrücker 5 und Hülse 13 des Pumpenzylinders 11 keine Verbindung über Schleppmittel. Die Rastnocke 14b und die innen am Betätigungsdrücker 5 anliegenden Federstege 16a, 16b verhindern mit geringer Klemmkraft daß der Betätigungsdrücker aus der Betätigungsendlage - wie dargestellt - in die Betätigungsausgangslage II, wie sie nachfolgend in Fig. 4a, 4b dargestellt ist, rutscht, ohne daß eine manuelle Betätigung des Betätigungsdrückers durch den Benutzer erfolgt. Der Rastnocken 14a der Hülse 13 ist im Rastloch 15a des Gehäuses 2 verrastet, dadurch ist sichergestellt, daß bei einer Bewegung des Betätigungsdrückers 5 aus der Betätigungsendlage I in die Betätigungsausgangslage II - also einer Bewegung entgegen einem Hub der Schubkolbenpumpe 10 - keine Bewegung des Pumpenzylinders 11 in die gleiche Richtung erfolgt. Wie aus der Schnittdarstellung der Fig. 3a ersichtlich, ist die Glasampulle 12, die in der Hülse 13 gehalten ist, durch einen Verschlußstopfen hermetisch verschlossen, der gleichzeitig als Kolben 21 der Schubkolbenpumpe 10 dient. Der Kolben 21 weist dabei eine Durchstichstelle für die Nadel 23 auf, welche in dem Schaft 22 angeordnet ist. Die Nadel 23 dient als Ausbringungskanal aus der Glasampulle 12 zu der Ausbringöffnung 4 im Gehäuse 2, sobald der Kolben 21 durchstochen wird. An dem Gehäuse 2 sind Grifflächen 3 angeformt, die zur Auflage jeweils eines Fingers, beispielsweise des Mittel- und des Zeigefingers dienen, während der Betätigungsdrücker mit dem Daumen betätigt wird, um einen Hub zu erzeugen. Wie aus Fig. 3b ersichtlich dient die Scherkante 19 am Gehäuse 2 nicht nur zur gehäuseseitigen Definition des ersten Anschlags 6 sondern auch dazu, das Druckpunktmittel 18 über die Scherkante 19 abzuscheren.

Durch manuelle Betätigung des Betätigungsdrückers 5 entgegen der Richtung eines Hubes, wie er zur Ausbringung einer Teilcharge, im vorliegenden Fall der Hälfte der Gesamtcharge, dient, wird die in den Figuren 4a und 4b dargstellte Betätigungsausgangslage II erreicht. Ein Ausbringungshub ist noch nicht erfolgt, kann nun jedoch jederzeit erfolgen. Noch immer ist die Lage der Hülse 13 bzgl. des Gehäuses 2 durch die in das Rastloch 15a eingreifende Rastnocke 14a der Hülse 13 gesichert, so daß auch weiterhin eine Bewegung des Pumpenzylinders 11 entgegen der Hubrichtung nicht möglich ist. In der Gleitführung des Betätigungsdrückers 5 im Gehäuse 2 ist ein zweiter Anschlag 7 ausgebildet, der sicherstellt, daß die dargestellte Betätigungsausgangslage II nicht über ein gewisses Toleranzmaß hinaus entgegen der Hubrichtung überfahren wird und sich so der Betätigungsdrücker 5 vom Gehäuse 2 trennt. In der Betätigungsausgangslage II greifen die hülsenseitigen Schleppmittel 16a, die durch einen während des Rückhubes überdrückbaren Federsteg mit einem Nocken gebildet werden, und die betätigungsdrückerseitigen Schleppmittel 17a so aneinander an, daß bei einer Hubbewegung des Betätigungsdrückers 5 der Pumpenzylinder 11 in Richtung auf die Ausbringöffnung 4 hin bewegt wird. Während einer solchen Bewegung wird die Verrastung zwischen dem Rastnocken 14a und dem Rastloch 15a gelöst. Die Hülse 13 gleitet dabei geführt von hülsenseitiger Führungsschiene 26 und gehäuseseitiger Führungsnut 25 verdrehsicher im Gehäuse 2 in Richtung auf die Ausbringöffnung 4. Nicht miteinander in Eingriff befinden sich die Schleppmittel für den zweiten Hub, die von dem Federsteg 16b und dem Mitnehmer 17b gebildet werden. An der Scherkante 19 des Gehäuses 2 liegen die hülsenseitigen Druckpunktmittel 18 an, die bei einer Betätigung der Schubkolbenpumpe zunächst abgeschert werden müssen. Die Druckpunktmittel 18 stellen sicher, daß eine Betätigung des Betätigungsdrückers nur dann zu einem Hub führt, wenn die Betätigung derart erfolgt, daß eine komplette Hubbewegung, die den Betätigungsdrücker 5 in die Betätigungsendlage I überführt, stattfindet.

Die Fig. 5a und Fig. 5b zeigen nunmehr die Stellung, die erreicht wird, wenn ein erster Hub stattgefunden hat. Der Pumpenzylinder 11 der Schubkolbenpumpe 10 wurde in Richtung auf die Ausbringöffnung 4 durch das Zusammenwirken von Federsteg 16a und Mitnehmer 17a bewegt. Die Nadel 23 hat den Kolben 21 durchstochen und bildet einen Ausbringungskanal zur Ausbringöffnung 4 des Gehäuses 2. Nach dem Durchstechen des Kolbens 21 durch die Nadel 23 gelangt der Schaft 22 in Anlage an den Kolben 21 und drückt ihn über den Weg eines Hubes hinweg in Richtung auf den Boden der Glasampulle 12, die in der Hülse 13 gehalten ist. Der Betätigungsdrücker 5 befindet sich wieder in Betätigungsendlage I, wobei sich der überdrückbare Federsteg 16a noch immer in Anlage zum Mitnehmer 17 befindet. Zur Sicherung des Pumpenzylinders 11 vor einer Bewegung entgegen der Hubrichtung und somit auch zur Sicherung eines Ansaugens von Luft in die Glasampulle 12 hinein verrastet die Rastnocke 14b in dem Rastloch 15b des Gehäuses 2. Ein Eingriff der Rastnocke 14a in das Rastloch 15a findet nicht mehr statt. Mit Erreichen der Betätigungsendlage gelangen die Druckpunktmittel 18 für den zweiten Hub in Anlage an die Scherkante 19 des Gehäuses. Ein Verhindern des Abscherens der Druckpunktmittel 18 für den zweiten Hub an der Scherkante 19 und das Überfahren der Betätigungsendlage I wird durch den ersten Anschlag 6 für den Betätigungsdrücker 5 an dem Gehäuse 2 sichergestellt. Damit ein zweiter Hub der Schubkolbenpumpe 10 stattfinden kann muß der Benutzer des Spenders zunächst den Betätigungsdrücker 5 manuell in die Betätigungsausgangslage II gebracht werden.

Die manuelle Betätigung des Betätigungsdrückers 5 zum Verbringen des Betätigungsdrückers aus der Betätigungsendlage I in die Betätigungsausgangslage II kann auch durch einen Kraftspeicher, insbesondere einen Federspeicher ersetzt werden; der Kraftspeicher muß in Betätigungsendlage I gespannt sein. Um ein bewußtes und gezieltes Verbringen des Betätigungsdrückers in die Betätigungsausgangslage II sicherzustellen, sind Haltemittel vorzusehen, die die gespannte Lage des Kraftspeichers lösbar festlegen, wobei ein Löseelement entweder am Gehäuse 2 oder am Betätigungsdrücker 5 vorzusehen sind, bei deren Betätigen ein Verbringen des Betätigungsdrückers 5 in die Betätigungsausgangslage II erfolgt. Der Kraftspeicher ist idealerweise schon vor dem ersten Verbringen des Betätigungsdrückers 5 in seine Betätigungsausgangslage II vorgespannt. Das Löseelement ist dann vorzugsweise selbst als Originalitätssicherung (d.h. nur offensichtlich, meist irreversibel beschädigt betätigbar) ausgebildet oder vor der Betätigung muß eine derartige Originalitätssicherung entfernt werden. Vorteilhaft ist es auch, wenn die Haltemittel nur vor dem ersten Betätigungshub an den Kraftspeicher angreifen, nach jedem Betätigungshub wird dann der Betätigungsdrücker 5 ohne weitere Betätigung des Löseelements wieder in die Betätigungsausgangslage II gebracht. In einfachster Ausbildung sind Haltemittel und Löseelement als sowohl mit Betätigungsdrücker 5 als auch mit Gehäuse 2 über Sollbruchstellen verbundener Abreißsteg ausgebildet. Dieser Abreißsteg muß vor dem Benutzen des Spenders vom Benutzer abgerissen werden, wonach durch die Wirkung des Kraftspeichers der Betätigungsdrücker 5 in Betätigungsausgangslage I verbracht wird.

Die Figuren 6a und 6b zeigen nunmehr den Betätigungsdrücker 5 in Betätigungsausgangslage II vor dem zweiten Hub. Bzgl. dem Gehäuse 2 in unveränderter Lage verbleiben die Hülse 13 und die darin gehaltene Glasampulle 12, wobei die Nadel 23, die in dem Schaft 22 angeordnet ist, weiterhin den Kolben 21 durchsetzt und somit einen Ausbringungskanal vom Ampulleninnenraum hin zur Ausbringungsöffnung 4 des Gehäuses 2 bildet. Die Lage der Hülse 13 im Gehäuse 2 wird durch die Rastnocke 14b, die in das Rastloch 15b eingreift, gesichert, während der Betätigungsdrücker 5 durch den Bediener manuell entgegen der Hubrichtung aus der in Fig. 5a, 5b dargestellten Betätigungsendlage I in die Betätigungsausgangslage II verbracht wird. Mit Erreichen der Betätigungsausgangslage II gelangt der Mitnehmer 17 in Eingriff mit dem Federsteg 16b, wodurch ein Schleppmittel für die Hülse 13 mit dem Betätigungsdrücker 5 gebildet wird. Die Druckpunktmittel 18 für den zweiten Hub befinden sich in Anlage mit der am Gehäuse 2 des Spenders 1 ausgebildeten Scherkante 19. Der Federsteg 16a gleitet innen anliegend in dem Gehäuse 2 und befindet sich nicht mehr in Eingriff. Gemäß einer vorzugsweisen, nicht dargestellten Ausführungsform ist es auch möglich, daß der überdrückbare Federsteg 16a, der während des ersten Hubes zum Mitnehmen der Hülse mit der Hubbewegung des Betätigungsdrückers 5 gedient hat, bei einem nachfolgenden Hub als Rastnocke zur Festlegung der Lage der Hülse 13 bzgl. dem Gehäuse 2 dient.

Die Fig. 7a und 7b zeigen nunmehr die Betätigungsendlage am Ende des zweiten - und im dargestellten Ausführungsbeispiel letzten - Hubes des Betätigungsdrückers 5 und der Schubkolbenpumpe 10.

Durch die zweite Betätigung des Betätigungsdrückers werden die Druckpunktmittel 18 (dargestellt in Fig. 6a, 6b) an der Scherkante 19 abgeschert. Dadurch wird sichergestellt, daß auch beim zweiten Hub der Betätigungsdrücker bis zum ersten Anschlag 6 bewegt wird, wodurch die Betätigungsendlage I erreicht ist. Der von der Nadel 23 durchstochene Kolben 21 taucht während der Hubbewegung des Pumpenzylinders 11, bestehend aus Glasampulle 12 und Hülse 13, soweit in die Ampulle 12 ein, daß sie den Grund der Ampulle 12 erreicht. Dabei wird die sich in der Glasampulle befindende zweite Teilcharge des auszubringenden Mediums über die Nadel 23 und die Ausbringöffnung 4 aus dem Spender ausgebracht. Der Pumpenzylinder 11 wird über den Mitnehmer 17, der an dem Betätigungsdrücker 5 ausgebildet, und in Wirkverbindung mit dem überdrückbaren Federsteg 16b ist, mitgeschleppt. Während der Betätigung des Betätigungsdrückers 5 wird auch die Rastverbindung zwischen der Hülse 13 und dem Gehäuse 2 des Spenders 1 gelöst. Ein erneutes Sichern der dargestellten Betätigungsendlage der Hülse 13 im Gehäuse 2 ist nicht mehr erforderlich, wenn der letzte Ausbringungshub durchgeführt wurde. Daher sind in dieser Lage keine Rastnocken, die an der Hülse angeformt sind, mehr im Eingriff mit Rastlöchern, die im Gehäuse 2 des Spenders 1 ausgebildet sind. In Betätigungsendlage befindet sich jedoch noch immer der Mitnehmer 17 in Berührung mit dem Federsteg 16b, ohne jedoch in der Lage zu sein, eine weitere Hubbewegung durchzuführen. Dies ist schon deshalb unmöglich, weil das Aufsitzen des Kolbens 21 am Boden der Glasampulle 12 eine Axialbewegung in Richtung auf die Ausbringungsöffnung des Gehäuses ebenso verhindert, wie die Anlage des Mitnehmers 17 an dem ersten Anschlag 6.

Der Spender 1 wird in der Regel mit dem Daumen am Betätigungsdrücker 5 und Zeige- und Mittelfinger an der Griffläche 3 betätigt. Spender, die in einem einzigen Hub oder in einer geringen Anzahl von Teilhüben eine Charge ausbringen und die bis zum ersten Betätigen des Spenders die Charge hermetisch abschließen, enthalten oft Medikamente oder Impfstoffe, die von dem Patienten zur sofortigen Anwendung bereitgehalten werden. So werden z.B. Medikamente auf die Nasenschleimhaut appliziert, weil sie von dort aus am schnellsten und am verträglichsten in den Blutkreislauf gelangen. Insbesondere Medikamente zur Bekämpfung von Migräne und anderen Kopfschmerzen werden so verabreicht. Die erfindungsgemäße Austragsvorrichtung gestattet es, die einzelnen Teilchargen mit großer Dosiergenauigkeit und Dosierzuverlässigkeit auszugeben; falls gewünscht auch in bemessenen, unterschiedlichen Mengen. Die Teilhübe sind wegen des bei der Erst-Betätigung zu durchlaufenden Leerweges unterschiedlich, was bei der Gestaltung insbesondere bei der Bemessung der überdrückbaren Federstege und der Lage der Mitnehmer zu berücksichtigen ist. Neben in die Nase (auf die Nasenschleimhäute) aufzubringende Medikamente eignet sich der Spender auch für Medikamente, die für Augen oder andere paarig oder mehrfach vorkommende Anwendungsorte bestimmt sind. Auch für mehrere aufeinanderfolgende Applikationen in Form von Spray oder anderer Ausgabeform, beispielsweise die Behandlung in Abständen einiger Minuten auf den gleichen Applikationsort, ist der Spender einsetzbar.

## Patentansprüche

1. Spender (1) mit manuell betätigbarer Ausbringeinrichtung (10) mit einem Pumpenzylinder (II), insbesondere zum zerstäubten Ausbringen einer Charge mit einem oder mehreren Hüben, mit einem Betätigungsdrücker (5) zur Betätigung der Ausbringeinrichtung (10), **dadurch gekennzeichnet, dass** an der Ausbringeinrichtung (10) und dem Betätigungsdrücker (5) zusammenwirkende Schleppmittel (16a, 16b) ausgebildet sind, wodurch der Pumpenzylinder bei einem Hub des Betätigungsdrückers (5) mitgeschleppt wird, wobei die Schleppmittel (16a, 16b) in einer Betätigungsausgangslage (2) in Wirkverbindung treten, und dass vor jedem Hub der Betätigungsdrücker (5) durch manuelle Betätigung aus einer Betätigungsendlage (I) ohne Hubbewegung des Pumpenzylinders in die Betätigungsausgangslage (II) bewegbar ist.

2. Spender nach Anspruch 1, **dadurch gekennzeichnet, dass** der Spender (1) ein Gehäuse (2) mit daran angeformter Grifffläche (3) zur Auflage wenigstens eines Fingers und mit einer Ausbringöffnung (4) aufweist, wobei der Betätigungsdrücker (5) relativbeweglich, insbesondere linearbeweglich zur Grifffläche (3) im Gehäuse (2) geführt ist.

3. Spender nach einem der vorhergehenden Ansprüche, **dadurch gekennzeichnet, dass** die Betätigungsendlage (I) durch einen die Hubbewegung des Betätigungsdrückers (5) begrenzenden ersten Anschlag (6) festgelegt ist.

4. Spender nach einem der vorhergehenden Ansprüche, **dadurch gekennzeichnet, dass** die Betätigungsausgangslage (II) durch einen die der Hubbewegung entgegengesetzte Bewegungsrichtung begrenzenden zweiten Anschlag (7) festgelegt ist.

5. Spender nach einem der vorhergehenden Ansprüche, **dadurch gekennzeichnet, dass** die Ausbringeinrichtung aus einer Schubkolbenpumpe (10) besteht, deren Pumpenzylinder (11) der Speicher für ein auszubringendes Medium ist und insbesondere aus einer zylindrischen Glasampulle (12) besteht, die in eine Hülse (13) eingesetzt ist.

6. Spender nach Anspruch 5, **dadurch gekennzeichnet, dass** an Pumpenzylinder (11) und Gehäuse (2) zusammenwirkende Rastmittel (14a, 14b, 15a, 15b) ausgebildet sind, die eine der Hubbewegung des Pumpenzylinders (11) entgegengesetzte Bewegung des Pumpenzylinders (11) verhindern, wobei die Rastmittel (14a, 14b, 15a, 15b) insbesondere bei Erreichen der Betätigungsendlage (I) des Betätigungsdrückers (5) ineinander greifen.

7. Spender nach einem der Ansprüche 5 oder 6, **dadurch gekennzeichnet**, daâ die Schleppmittel (16a, 16b) derart ausgebildet sind, dass die Schleppverbindung bei einer der Hubbewegung entgegengesetzten Bewegung gelöst wird.

8. Spender nach einem der Ansprüche 5 bis 7, wobei zwischen Pumpenzylinder (11) und Gehäuse (2) wirkende Rastmittel (14a, 15a, 14b, 15b) zum Verhindern einer Bewegung des Pumpenzylinders entgegen der Hubbewegung ausgebildet sind, wobei zwischen Betätigungsdrücker (5) und Pumpenzylinder (11) wirkende Schleppmittel (16a, 16b) zum Mitschleppen des Pumpenzylinders während der Hubbewegung des Betätigungszylinders ausgebildet sind, **dadurch gekennzeichnet, daß** die pumpenzylinderseitigen Teile der Schleppmittel so ausgebildet sind, daß sie bei einer nachfolgenden Betätigung des Spenders (1) auch als pumpenzylinderseitige Rastmittel dienen.

9. Spender nach einem der vorstehenden Ansprüche, **dadurch gekennzeichnet, daß** die Bewegung des Betätigungsdrückers (5) aus der Betätigungsendlage (I) in die Betätigungsausgangslage (II) manuell erfolgt.

10. Spender nach einem der Ansprüche 1 bis 13, **dadurch gekennzeichnet, daß** die Bewegung des Betätigungsdrückers (5) aus der Betätigungsendlage (I) in die Betätigungsausgangslage (II) mittels eines Kraftspeichers erfolgt, wobei der Kraftspeicher vorzugsweise in Betätigungsendlage (I) durch Haltemittel vorgespannt gehalten ist und die Haltemittel durch Betätigen eines Löseelements lösbar sind, wodurch eine Bewegung des Betätigungsdrückers (5) von der Betätigungsendlage (I) in die Betätigungsausgangslage (II) erfolgt und insbesondere bei oder vor dem ersten Betätigen des Löseelements eine Originalitätssicherung irreversibel zerstört wird.

11. Spender nach Anpsruch 10, **dadurch gekennzeichnet, daß** die Haltemittel lediglich vor dem ersten Betätigen des Löseelements den Kraftspeicher vorgespannt halten, wobei das Löseelement und die Haltemittel insbesondere als zwischen Gehäuse (2) und Betätigungsdrücker (5) beidseitig über Sollbruchstelle angeordneter Abreißsteg ausgebildet sind.

12. Spender nach einem der vorhergehenden Ansprüche, **dadurch gekennzeichnet, daß** der Betätigungsdrücker einen rechteckigen Querschnitt aufweist und daß vorzugsweise das Gehäuse (2) in dem Bereich des Betätigungsdrückers einen rechteckigen Querschnitt aufweist.

13. Spender nach einem der vorhergehenden Ansprüche, **gekennzeichnet durch** Druckpunktmittel (18), die Sollbruchmaterialbrücken enthalten, die zwischen dem Gehäuse (2) und dem Pumpenzylinder (11) wirksam sind.

14. Spender nach einem der vorhergehenden Ansprüche, **dadurch gekennzeichnet, daß** die als Schubkolbenpumpe ausgebildete Ausbringeinrichtung einen Kolben (21) aufweist, der von einem durchstechbaren, einen Verschluß für den Pumpenzylinder der Schubkolbenpumpe bildenden Verschlußstopfen gebildet ist, der vorzugsweise von einem im Ausbringungsabschnitt gelagerten Schaft (22) unreversierbar in den Pumpenzylinder hineindrückbar und von einer in den Schaft (22) gehaltenen Nadel (23) durchstechbar ist.

## Claims

1. Dispenser (1) with manually operable discharge device (10) with a pump cylinder (11), particularly for the atomized discharge of a charge with one or more strokes, with an operating presser (5) for operating the discharge device (10), **characterized in that** on the discharge device (10) and the operating presser (5) are formed cooperating drag means (16a, 16b), so that during a stroke of the operating presser (5) the pump cylinder is carried along, the drag means (16a, 16b) being in operative connection in an operating starting position (2), and that prior to each stroke the operating presser (5) can be moved by manual operation from an operating end position (I) without stroke movement of the pump cylinder into the operating starting position (II).

2. Dispenser according to claim 1, **characterized in that** the dispenser (1) has a casing (2) with a gripping surface (3) shaped thereon for supporting at least one finger and with a discharge opening (4), the operating presser (5) being guided in relatively movable manner, in particular linearly movable to the gripping surface (3) in the casing (2).

3. Dispenser according to one of the preceding claims, **characterized in that** the operating end position (I) is fixed by a first stop (6) limiting the stroke movement of the operating presser (5).

4. Dispenser according to one of the preceding claims, **characterized in that** the operating starting position (II) is fixed by a second stop (7) limiting the movement direction in opposition to the stroke movement.

5. Dispenser according to one of the preceding claims, **characterized in that** the discharge device comprises a thrust piston pump (10), whose pump cylinder (11) is the reservoir for a medium to be discharged and in particular comprises a cylindrical glass ampoule (12) inserted in a sleeve (13).

6. Dispenser according to claim 5, **characterized in that** cooperating locking means (14a, 14b, 15a, 15b) are formed on the pump cylinder (11) and casing (2) and prevent a movement of the pump cylinder (11) in opposition to its stroke movement, the locking means (14a, 14b, 15a, 15b) particularly interengaging when the operating presser (5) reaches the operating end position (I).

7. Dispenser according to claim 5 or 6, **characterized in that** the drag means (16a, 16b) are constructed in such a way that the drag connection is released during a movement in opposition to the stroke movement.

8. Dispenser according to one of the claims 5 to 7, in which locking means (14a, 15a, 14b, 15b) acting between the pump cylinder (11) and casing (2) are constructed for preventing a pump cylinder movement counter to the stroke movement and in which drag means (16a, 16b) acting between the operating presser (5) and pump cylinder (11) are constructed for carrying along the pump cylinder during the stroke movement of the operating cylinder, **characterized in that** the pump cylinder-side parts of the drag means are constructed in such a way that they also serve as pump cylinder-side locking means during a following operation of the dispenser (1).

9. Dispenser according to one of the preceding claims, **characterized in that** the movement of the operating presser (5) from the operating end position (I) into the operating starting position (II) takes place manually.

10. Dispenser according to one of the claims 1 to 8, **characterized in that** the movement of the operating presser (5) from the operating end position (I) into the operating starting position (II) takes place by means of a force reservoir, the force reservoir preferably being kept pretensioned in the operating end position (I) by holding means, which are releasable by the operation of a release member, so that a movement of the operating presser (5) from the operating end position (I) to the operating starting position (II) takes place and particularly where during or prior to the first operation of the release member, a tamper-proof seal is irreversibly destroyed.

11. Dispenser according to claim 10, **characterized in that** the holding means only keep the force reservoir pretensioned prior to the first operation of the release member, the release member and the holding means being more particularly constructed as a tear-off web connected on either side by means of predetermined breaking points to the casing (2) and operating presser (5).

12. Dispenser according to one of the preceding claims, **characterized in that** the operating presser has a rectangular cross-section and that preferably the casing (2) has a rectangular cross-section in the area of the operating presser.

13. Dispenser according to one of the preceding claims, **characterized in that** pressure point means (18) are provided containing predetermined breaking material bridges acting between the casing (2) and the pump cylinder (11).

14. Dispenser according to one of the preceding claims, **characterized in that** the discharge device constructed as a thrust piston pump has a piston (21), which is formed by a perforatable plug forming a closure for the pump cylinder of the thrust piston pump and which is preferably irreversibly pressable into the pump cylinder by a shaft (22) mounted in the discharge section and is perforatable by a needle (23) held in the shaft (22).

## Revendications

1. Distributeur (1) avec un dispositif d'expulsion (10) pouvant être actionné à la main, présentant un cylindre de pompe (11), notamment pour l'expulsion d'une charge pulvérisée par une ou plusieurs courses, avec un poussoir d'actionnement (5) pour l'actionnement du dispositif d'expulsion (10), **caractérisé en ce que** des moyens d'entraînement (16a, 16b) agissant conjointement sont réalisés sur le dispositif d'expulsion (10) et sur le poussoir d'actionnement (5), le cylindre de pompe étant entraîné pendant une course du poussoir d'actionnement (5), les moyens d'entraînement (16a, 16b) dans une position initiale d'actionnement (2) se mettant en contact actif, et **en ce que**, avant chaque course, le poussoir d'actionnement (5) est déplaçable par un actionnement manuel à partir d'une position terminale d'actionnement (I) dans la position initiale d'actionnement (II) sans aucun mouvement de course du cylindre de pompe.

2. Distributeur d'après la revendication 1, **caractérisé en ce que** le distributeur (1) présente un boîtier (2) avec une surface de préhension (3) ajoutée par moulage, prévue pour poser au moins un doigt et avec un orifice de décharge (4), le poussoir d'actionnement (5) étant guidé dans le boîtier (2) de manière à pouvoir effectuer des mouvements relatifs, notamment des mouvements linéaires par rapport à la surface de préhension (3).

3. Distributeur d'après une des revendications précédentes, **caractérisé en ce que** la position terminale d'actionnement (I) est déterminée par un premier arrêt (6) délimitant le mouvement de course du poussoir d'actionnement (5).

4. Distributeur d'après une des revendications précédentes, **caractérisé en ce que** la position initiale d'actionnement (II) est déterminée par un deuxième arrêt (7) délimitant la direction de mouvement opposée à la direction de course.

5. Distributeur d'après une des revendications précédentes, **caractérisé en ce que** le dispositif d'expulsion consiste en une pompe à piston-poussoir (10), dont le cylindre de pompe (11) est le réservoir pour une substance à décharger, étant notamment constitué d'une ampoule cylindrique en verre (12), qui est insérée dans une douille (13).

6. Distributeur d'après la revendication 5, **caractérisé en ce que** sur le cylindre de pompe (11) et sur le boîtier (2) sont réalisés des moyens d'enclenchement (14a, 14b, 15a, 15b) agissant conjointement, qui empêchent un mouvement du cylindre de pompe (11) en direction opposée au mouvement de course du cylindre de pompe (11), les moyens d'enclenchement (14a, 14b, 15a, 15b) s'engageant l'un dans l'autre notamment quand le poussoir d'actionnement (5) atteint la position terminale d'actionnement (I).

7. Distributeur d'après une des revendications 5 ou 6, **caractérisé en ce que** les moyens d'entraînement (16a, 16b) sont réalisés de manière que le joint à entraînement se décompose pendant un mouvement en direction opposée à la direction du mouvement de course.

8. Distributeur d'après une des revendications de 5 à 7, où des moyens d'enclenchement (14a, 15a, 14b, 15b) qui produisent leur action entre le cylindre de pompe (11) et le boîtier (2) sont réalisés de manière à pouvoir empêcher un mouvement du cylindre de pompe orienté dans une direction opposée à la direction de course, des moyens d'entraînement (16a, 16b), qui produisent leur action entre un poussoir d'actionnement (5) et le cylindre de pompe (11), étant réalisés de manière à pouvoir entraîner le cylindre de pompe pendant le mouvement de course du cylindre d'actionnement, **caractérisé en ce que** les éléments des moyens d'entraînement, du côté du cylindre de pompe, sont réalisés de manière à servir aussi en tant que moyens d'enclenchement du côté du cylindre de pompe pendant un actionnement ultérieur du distributeur (1).

9. Distributeur d'après une des revendications précédentes, **caractérisé en ce que** le mouvement du poussoir d'actionnement (5) à partir de la position terminale d'actionnement (I) dans la position initiale d'actionnement (II) est effectué manuellement.

10. Distributeur d'après une des revendications de 1 à 8, **caractérisé en ce que** le mouvement du poussoir d'actionnement (5) à partir de la position terminale d'actionnement (I) dans la position initiale d'actionnement (II) est effectué au moyen d'un accumulateur d'énergie, l'accumulateur d'énergie est maintenu sous précontrainte de préférence en position terminale d'accumulation (I) par des moyens de retenue et les moyens de retenue étant désenclenchables par l'actionnement d'un élément de désenclenchement, ce qui produit un mouvement du poussoir d'actionnement (5) de la position terminale d'actionnement (I) dans la position initiale d'actionnement (II) et la destruction irréversible d'un dispositif protecteur de l'état d'origine notamment pendant ou avant le premier actionnement de l'élément de désenclenchement.

11. Distributeur d'après la revendication 10, **caractérisé en ce que** les moyens de retenue maintiennent l'accumulateur d'énergie en état de précontrainte uniquement avant le premier actionnement de l'élément de désenclenchement, l'élément de désenclenchement et les moyens de retenue étant notamment réalisés en tant que nervure de rupture disposée entre le boîtier (2) et le poussoir d'actionnement (5), des deux côtés au-dessus de la section de rupture intentionnellement prévue.

12. Distributeur d'après une des revendications précédentes, **caractérisé en ce que** le poussoir d'actionnement présente une section transversale rectangulaire et **en ce que** de préférence le boîtier (2) présente une section transversale rectangulaire dans le domaine du poussoir d'actionnement.

13. Distributeur d'après une des revendications précédentes, **caractérisé par** des moyens à point de poussée (18), qui comprennent des ponts à rupture intentionnellement prévue, qui agissent dans le domaine entre le boîtier (2) et le cylindre de pompe (11).

14. Distributeur d'après une des revendications précédentes, **caractérisé en ce que** le dispositif d'expulsion réalisé comme pompe à piston-poussoir présente un piston (21), qui se constitue d'un bouchon pouvant être percé et formant une fermeture pour le cylindre de pompe de la pompe à piston-poussoir, le bouchon pouvant de préférence être enfoncé irrévocablement dans le cylindre de pompe par une tige (22) logée dans la section d'expulsion et pouvant être percé par une aiguille (23) tenue dans la tige (22).
